# EUROPEAN PATENT APPLICATION

(11) **EP 2 105 388 A1**
(43) Date of publication of application: **30.09.2009**
(21) Application number: 08016459.3
(22) Date of filing: 18.09.2008
(51) Int. Cl.: B65D 51/24, B65D 81/36

(54) **Lid for beverage container with cavity for a figure or toy**

(30) Priority: 25.03.2008 MX PA08000098
(71) Applicant: Golden Link Inc., Washingtonville NY 10992 (US)
(72) Inventor: Waaland, Jeffrey L., Washingtonville New York 10992 (US)
(74) Representative: Isern-Jara, Nuria

(57) **Abstract**

This invention refers to a "lid for a beverage container" with cavity for a figure or toy, which proposes a lid created with a hole to insert a straw (3) and a central cavity (4) allowing the introduction and extraction of a figure (5) which, once inserted or without insertion, forms a secure and complete incorporation over the cup or container, so that the lid and the figure can be presented and sold to the consumer as a single product, while allowing the unique function that the figure/toy can be separated from the lid creating an independent figure/toy. This independent figure/toy can also be used as a pencil topper or finger puppet.

## Description

### PURPOSE OF THE INVENTION.

This invention, which consists of a lid for a beverage container, proposes a lid made with an opening to insert a straw and a central cavity which allows a figure to be introduced and removed which, once inserted or without insertion, forms a safe and complete incorporation over the cup or container, so that the lid and the figure can be presented and sold to the consumer as a single product. The lid is easy to assemble and disassemble thanks to its mechanism, which will be amply explained later, thus achieving an excellent function not currently on the market and therefore completely unknown to the consumer public, since by means of this proposal the lids can now cover cups or containers with articles which facilitate the sale of many products with figures (under license and generic), such as cup and cover sets, with the sole and exclusive advantage that the figure can be separated from the cover, permitting the novelty of having an independent toy as a prize or profit.

### ANTECEDENTS OF THE INVENTION.

The consumer public, within the human environment, has tried to find a way to satisfactorily obtain an effective and entertaining use for the different kinds of lids currently existing for beverage containers, and to be able to differentiate between the large variety of lids available in the business, but unfortunately it has not been possible to meet the customer's needs since only normal lids are available, the purpose of which is to aid in retaining liquids in the container, failing to comply with the entertaining and different use which could be provided by a new and functional beverage container lid, since the antecedents to the present invention include cup covers formed in conjunction with the lid by means of a process known as "Blowmold" (molding based on blowing), by means of which the cover and the lid are physically incorporated and cannot be separated. Another similar, although different, product is that known "Bellywashers", consisting of covers applied to a screw-type lid placed in a plastic bottle for boat-style drinking. Therefore, today we present a new proposal, more advanced, entertaining, efficient and different from the lids already known, characterized basically by their special form and configuration which gives the lid a strange and individual aspect for assembly to the container, accepting liquids and for inserting figures into the lid cavity, on utilization in any type of container.

### DESCRIPTION OF THE INVENTION.

This invention, which consists of a lid for a beverage container, proposes a lid created with a central cavity allowing the introduction and extraction of a figure which, once inserted or without insertion, forms a safe and complete incorporation over the cup or container, which can then be presented and sold to the consumer as a single product. Once presented to the customer as a single product (cup/lid/topper), the invention allows the "topper" figure to be detached from the lid, creating an independent toy/premium for the consumer.

The lid consists of a groove placed at the base of the lower part cavity, allowing the base of the cover to be permanently affixed to the lid, thus forming a secure connection. Opposite, a smaller edge appears at the base of the cover to balance said lid.

A "rim" is formed on the lid covering the upper part of the cavity. The reason for this is to have a fold, thus creating another point of connection which allows the base of the lid to be attached and creates a firm, flat base larger than the cavity for the real cover, to serve as support in the upper part. Also, the reverse is designed for incorporation to the base of the cover and with the lid at the upper part of the base, ensuring secure incorporation to the edge of the lid.

The design of the lid has also been added in order for this to be more up to date, and for the lid to be used with paper cups. Formerly, all covers or lids had been created for utilization with and incorporation to (and even selling with) plastic cups. The latest and most up to date lid includes the same basic design previously used to cover plastic cups, but with the following additional features:
It has been created for production by means of a "Thermoform" type process, as opposed to the molding by injection process used for plastic cup lids.
A design type cover similar to that known as dome or vault-type cover used for coffee. This design has 2 added benefits in that the dome style allows the toy/topper to sit high above the cup for superior presentation, while also allowing the cavity portion of the lid not to interfere with the drink/ice fill capacity, which would occur if the bottom of the cavity went below the top "fill line" of the cup. This provides the cover with a certain height or elevation so that it has a space or cavity designed for the liquid not to go over a certain level.
A closing mechanism on the lower inside edge permitting additional "locking" feature for attachment and adherence to the paper cup.

### BRIEF DESCRIPTION OF THE FIGURES.

In order to better understand the features of the present invention and to complement the present description, attached hereto as an integral part of same are drawings of an illustrative nature which, without limitation, represent the following:

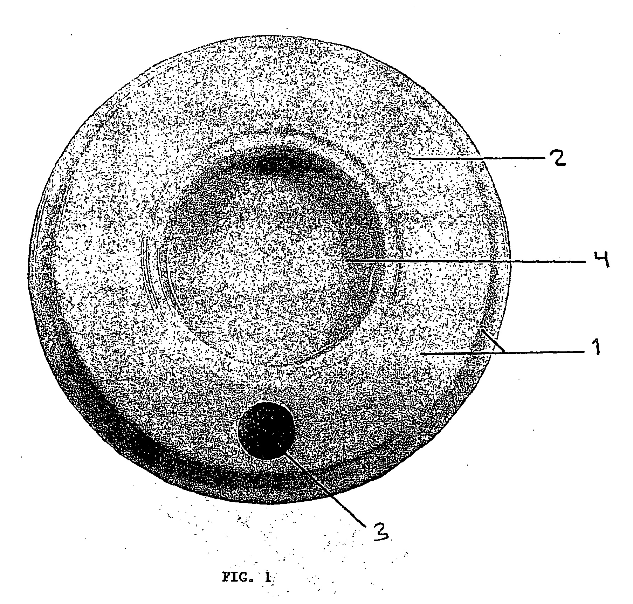

**Figure 1****.-** Shows the lid from above.

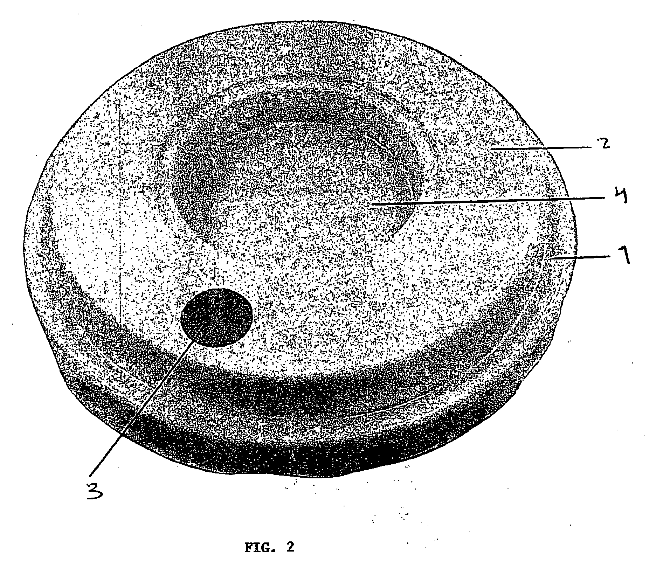

**Figure 2****.-** Shows the lid from above and facing.

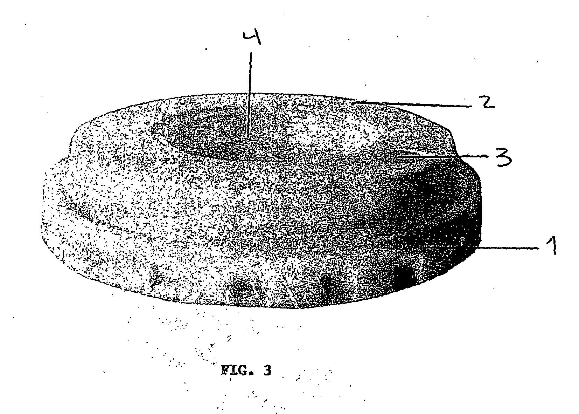

**Figure 3****.-** Shows the lid from the side.

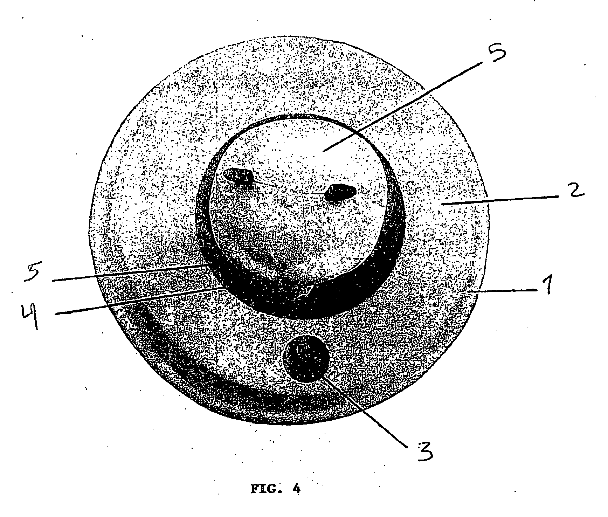

**Figure 4****.-** Gives a detailed view of the lid from above, with a decorative figure attached from the top.

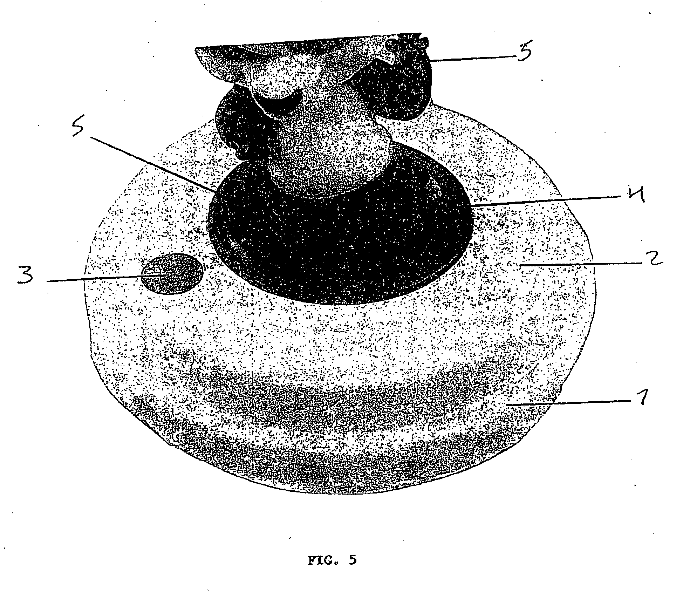

**Figure 5****.-** Shows in detail an upper and facing view of the lid with a decorative figure attached to same from the top.

### DETAILED DESCRIPTION OF THE MODEL IN QUESTION.

The present invention refers to a Lid (1) for bottling beverages, characterized by consisting of a Base (2) with a Hole for a straw (3) and a Cavity for a Figure (4) into which a Figure can be introduced or extracted (5) which will subsequently be attached to the Base (2) of the Lid (1).

### List of reference:

Lid (1)
Base (2)
Hole for straw (3)
Cavity for Figure (4)
Figure (5)

Having sufficiently described the invention, we consider it a novelty and therefore claim the contents of the following clauses as our exclusive property:

## Claims

1. Lid **characterized by** having a hole for inserting a straw and a central cavity permitting the introduction and extraction of a figure which, once inserted or without insertion, forma a secure and complete incorporation over the cup or container, so that the lid and the figure can be presented and sold to the consumer as a single product.

2. Lid in accordance with claim 1, **characterized** because it has a groove placed at the base of the lower part of the cavity, permitting the base of the cavity to be permanently attached to the lid, thus forming a secure connection.

3. Lid in accordance with claim 1, **characterized** because the lid consists of a card placed on the lower part of the base of the cover, thus creating a "Card/Cover" type product.

4. Lid in accordance with claim 1, **characterized** because the base of the cover is separated from the figure, thus creating an independent figure not incorporated to the base of the lid.

5. Lid in accordance with claim 1, **characterized** because the topper consists of two pieces, the first is a base with a "post", of which this piece is used to attach to the topper lid, and the second is the actual figure/premium which slides over the post to create the same presentation of a single product to the consumer. The figure once extracted becomes an independent toy that can also be used as a "pencil" topper or "finger puppet".

6. Lid in accordance with claim 1, **characterized** because the independent figure has an additional attachment to the bottom of the "topper base" such as a "stamp", "game piece", "sound activated chip", or other addition feature that enhances the topper uses.

7. Lid in accordance with claim 1, **characterized** because it can be used as a special collectable sports figure, especially useful with added "bobble head" feature of the figures/toppers.

8. Lid in accordance with claim 1, **characterized** because utilization for paper cups by means of a "Thermoform" type process, as opposed to the holding by injection process utilized for lids in plastic cups.

9. Lid in accordance with claim 1, **characterized** because the design provides the cover with a certain height or elevation so that it has a space or cavity designed for the liquid not to go over a certain level.

10. Lid in accordance with claim 1, **characterized** because it consists of a closing mechanism on the lower inside edge which allows it to be secured and attached to the paper cup.
